## Europäisches Patentamt

## European Patent Office

## Office européen des brevets

(19)

(11) Publication number: **0 018 967**
**B1**

# EUROPEAN PATENT SPECIFICATION

(12)

(45) Date of publication of patent specification: **07.07.82**

(21) Application number: **79900093.0**

(22) Date of filing: **20.11.78**

(86) International application number:
**PCT/US78/00159**

(87) International publication number:
**WO 79/00475 26.07.79 Gazette 79/15**

(51) Int. Cl.³: **G 01 N 33/54,**
**A 61 K 39/00, C 12 Q 1/42**

(54) Composition and a diagnostic method for the detection of prostatic cancer.

(30) Priority: **04.01.78 US 866918**

(43) Date of publication of application:
**26.11.80 Bulletin 80/24**

(45) Publication of the grant of the patent:
**07.07.82 Bulletin 82/27**

(84) Designated Contracting States:
**CH DE FR GB SE**

(56) References cited:
**US - A - 3 932 221**
**US - A - 4 012 285**
**US - A - 4 016 043**
**US - A - 4 046 634**

**Cancer Treatment Reports, VOL. 61, No. 2, issued March/April 1977, Chu et al, Enzyme Markers In Human Prostatic Carcinoma, pages 193—200.**

(73) Proprietor: **Research Corporation**
**405 Lexington Avenue**
**New York, N.Y. 10017 (US)**

(72) Inventor: **CHU, Tsann, Ming**
**117 Old Orchard Road**
**Williamsville, NY 14221 (US)**
Inventor: **WANG, Ming, Chang**
**79 Troy View Road**
**Williamsville, NY 14221 (US)**

(74) Representative: **Rasper, Joachim, Dr.**
**Bierstadter Höhe 22**
**D-6200 Wiesbaden (DE)**

(56) References cited:
**The New England Journal of Medicine, VOL. 297, No. 25, issued 22 December 1977, Foti et al, Detection of Prostatic Cancer by Solid-phase Radioimmunoassay of Serum Prostatic Acid Phosphatase, pages 1357—1361.**

**Journal of Immunology, VOL. 93, issued 1964, Shulman et al, The Detection of Prostatic Acid Phosphatase by Antibody Reactions In Gel Diffusion, pages 474—480.**

Courier Press, Leamington Spa, England.

# 0018967

Composition and a diagnostic method for the detection of prostatic cancer

Technical Field

This invention relates to a diagnostic reagent and method for the immunochemical detection of serum prostatic acid phosphatase. More particularly, this invention relates to a purified isoenzyme antigen and antibodies specific thereto which are suitable for use in prostatic cancer detection by laboratory methods.

Background Art

Acid phosphatases are enzymes capable of hydrolyzing phosphate monoesters in an acid medium at around pH 5. Human acid phosphatases are normally found in virtually all human tissues, e.g., the prostate, bladder, kidney, liver, spleen, platelets, erythrocytes, brain, bone marrow, lung, intestine and testicles. Originally believed to be a single enzyme, human acid phosphatases all share the common enzymatic property of hydrolyzing phosphate esters in an acid medium but are now known to exist in a plurality of at least fifty biochemically distinct forms. For example, erythrocyte acid phosphatase is a protein having a molecular weight of about 20,000 daltons while prostate acid phosphatase is a glycoprotein having a molecular weight of about 100,000 daltons and which, in samples extracted from cancerous prostate tissue, exists in at least eight electrophoretically separable isoenzyme forms as recently reported by Chu et al. in Cancer Treatment Reports *61:* 193 (1977).

The elevation of serum acid phosphatase activity in patients having metastasized prostate carcinoma was first reported by Gutman et al. in J. Clin. Invest. *17:* 473 (1938). In cancer of the prostate, prostatic acid phosphatase is released from the cancer tissue into the blood stream with the result that the total serum acid phosphatase level greatly increases above normal values. Numerous studes of this enzyme and its relation to prostatic cancer have been made since that time, e.g., see the review by Yam in Amer. J. Med. *56:* 604 (1974). However, the measurement of serum acid phosphatase by conventional spectrophotometric methods often fails to detect prostatic cancer in its early stages. In general, the activity of serum acid phosphatase is elevated in about 65—90% of patients having carcinoma of the prostate with bone metastasis; in about 30% of patients without roentgenological evidence of bone metastasis; and in about only 5—10% of patients lacking clinically demonstrable metastasis.

Shinowara et al. reported a method for determining serum acid phosphatase in J. Biol. Chem. *142:* 921 (1942) which employs glycerophosphate as the enzyme substrate and spectrophotometrically measures the product of enzyme reaction, phosphate ion, as a colored phosphomolybdate complex. Since serum normally contains an appreciable quantity of phosphate ions and that released by the acid phosphatase is small in relation thereto, it is necessary to carry out a blank determination of normal serum phosphate content for each sample being tested and to carefully determine the difference in total phosphate content between the blank control and the actual test containing additional enzyme-released phosphate.

Babson, U.S. Patent 3,002,893 describes a method for determining serum acid phosphatase which emplys the buffered salt of an alpha-naphthyl phosphate as the enzyme substrate. While initially believed specific for the detection of acid phosphatase released by the prostate gland, subsequent investigators have reported difficulties in realizing this objective. For example, fairly cumbersome inhibition techniques, e.g., with tartrate or formalin, must generally be employed to satisfactorily mask interfering activity of erythrocyte acid phosphatase. Furthermore, this substrate has about the same activity for platelet acid phosphatase as for phostatic acid phosphatase, which cannot be eliminated by the use of such inhibition techniques.

Roy et al., U.S. Patent 3,823,071 describes the use of water-soluble metal salts of thymolphthalein monophosphate as substrates which exhibit improved specificity in comparison with beta-glycerolphosphate and alpha-naphthylphosphate in the detection of prostatic acid phosphatase. However, Ewen et al. have reported in Clin. Chem. *22:* 627 (1976) that thymolphthalein monophosphate is a non-specific substrate for prostatic acid phosphatase, cross-reacting with a large number of other acid phosphatases.

The aforementioned attempts to develop a specific test for prostatic acid phosphatase have met with only limited success because techniques which rely on enzyme activity on a so-called "specific" substrate cannot take into account other biochemical and immunochemical differences among the many acid phosphatases which are unrelated to enzyme activity of prostate origin. In the case of isoenzymes, i.e. genetically defined enzymes having the same characteristic enzyme activity and a similar molecular structure but differing in amino acid sequences and/or content and therefore immunochemically distinguishable, it would appear inherently impossible to distinguish different isoenzyme forms merely by the choice of a particular substrate. It is therefore not surprising that none of these prior art methods is highly specific for the direct determination of prostatic acid phosphatase activity; e.g. see Cancer *5:* 236 (1952); J. Lab. Clin. Med. *82:* 486 (1973); Clin. Chem. Acta. *44:* 21 (1973); and J. Physiol. Chem. *356:* 1775 (1975).

In addition to the aforementioned problems of non-specificity which appear to be inherent in many of the prior art reagents employed for the detection of prostate acid phosphatase, there have been reports of elevated serum acid phosphatase associated with other diseases, which further complicates the problem of obtaining an accurate clinical diagnosis of prostatic cancer. For example, Tuchman et al. in Am. J. Med. *27:* 959 (1959) have noted that serum acid phosphatase levels appear to be elevated in patients with Gaucher's disease.

Due to the inherent difficulties in developing a "specific" substrate for prostate acid phosphatase, several researchers have developed immunochemical methods for the detection of prostate acid phosphatase. However, the previously reported immunochemcial methods have drawbacks of their own which have precluded their widespread acceptance. For example, Shulman et al., in Immunology *93:* 474 (1964) described an immunodiffusion test for the detection of human prostate acid phosphatase. Using antisera prepared from a prostatic fluid antigen obtained by rectal massage from patients with prostatic disease, no cross-reactivity precipitin line was observed in the double diffusion technique against extracts of normal kidney, testicle, liver and lung. However, this method has the disadvantages of limited sensitivity, even with the large amounts of antigen employed, and of employing antisera which may cross-react with other, antigenically unrelated serum protein components present in prostatic fluid.

Foti et al. reported in Cancer Research *35:* 2446 (1975) the use of radioimmunoassay (RIA) techniques for measuring serum acid phosphatase. Using a similarly obtained prostatic fluid antigen for the production of antisera, preliminary clinical trials showed that patients having advanced prostatic cancer exhibited elevated acid phosphatase levels. The RIA technique is extremely sensitive, but takes several days to perform and requires sophisticated equipment and highly trained personnel. This method has the further inherent disadvantages of a short useful shelf life of only about one month for the reagents employed due to the brief half-life of radioactive iodine 125 and of a false positive rate of about 14% as reported recently in Clin. Chem. *23:* 95—99 (1977).

Foti in the New England Journal of Medicine, Vol. 297, No. 25, pages 1357—1361, has compared a solid-phase radioimmunoassay with a standard enzymatic spectrophotometric method in the measurement of serum prostatic acid phosphatase, but he did not realize that there is a difference in serum acid phosphatase at the molecular level from patients with prostatic carcinoma and normal men or patients having other diseases.

SCHUURS In US patent 4,016,043 reports a method for detection and determination of antigen-antibody reactions, including the step of covalently bonding antibodies to a water-insoluble support, and he has shown that his method is especialy useful for diagnostic testing for hepatitis or rubella antibodies.

Thus, there is still a need for simple, reliable, sensitive and specific reagents and techniques to measure prostatic acid phosphatase with acceptable diagnostic accuracy and without the aforementioned difficulties of the prior art. The present invention fills such needs.

Disclosure of the Invention

It is a general object of the present invention to provide an improved diagnostic reagent suitable for the immunochemically specific detection of cancerous prostatic serum phosphatase isoenzyme patterns.

Another object of this invention is to provide a rapid and simple method for the early detection of prostatic cancer.

A further object of the present invention is to provide a highly specific and sensitive immunochemical technique and reagents useful in the early detection of prostatic cancer.

Best Mode for Carrying Out the Invention

Briefly, the above and other objects, features and advantages of the present invention are attained in one aspect thereof by providing immunoprecipitating antisera which are highly specific to cancerous prostatic acid phosphatase isoenzyme patterns and which do not immunochemically cross-react with acid phosphatases originating from other tissues or against the normal human prostatic acid phosphatase isoenzyme forms.

In a second aspect of the present invention, there is provided an immunochemical method for the detection of cancerous prostatic acid phosphatase isoenzyme patterns which exhibits high sensitivity, good specificity and substantially no false positive results in the detection of prostatic cancer.

According to the present invention, antigenic preparations from cancerous human prostate tissue or fluid are purified to obtain a purified prostatic acid phosphatase preparation consisting essentially of the isoenzymes associated with prostatic cancer having an isoelectric point (pI) of 4.5—5.5, especially 4.5—5.0. These antigenic preparations are employed for immunological vaccination and diagnostic procedures, particularly for immunoprecipitin testing. While not wishing to be bound by any theory of the present invention, it is postulated herein that greatly improved antigenic preparations are obtainable by isolating and purifying prostatic acid phosphatase from cancerous human prostate, so that the characteristic isoenzyme pattern thereof corresponds to that associated with carcinoma of the prostate rather than the isoenzyme pattern of normal prostate tissue or fluid, which have generally been employed in the prior art.

Furthermore, it has now been found that the human prostatic acid phosphatase isoenzymes associated with prostatic cancer appear to have different sites for antibody binding and for enzyme activity. Therefore, immunoprecipitin antigen-antibody complexes such as are obtained in immunoelectrophoresis continue to exhibit the enzyme activity characteristic of prostatic acid phosphates so that a combination of immunological and enzymatic techniques can now be applied for the detection of acid phosphatases, thereby greatly enhancing the combined sensitivity and specificity of previous methods. This is the basis for the development of both the counterimmuno- electrophoresis technique as well as the solid-phase fluorescent immunoassay of this clinically important enzyme.

Anti-prostatic acid phosphatase not only separates prostatic acid phosphatase from other phosphatases and serum proteins, but also stabilizes the enzyme. Subsequently, the activity of prostatic acid phosphatase is measured by the hydrolytic product, which can be quantitated with great sensitivity. Preferably, either the prostatic acid phosphatase substrate or its corresponding hydrolytic product is fluorogenic, so that the enzyme activity can be readily determined with great sensitivity by spectrofluorometric techniques. Suitable such substrates are well known in the art and described, e.g. by M. Roch in "Fluorometric Assay of Enzymes" appearing in Methods of Biochemical Analysis *17:* 189—285 (1969) and by Lowry et al. in "A Flexible System of Enzymatic Analysis" published in New York by Academic Press, Inc. (1972). Presently preferred are substrates whose hydrolytic products are chromogenic while the unhydrolyzed substrate is not. Such compounds are likewise well known in the art and include but are not limited to alpha-naphthyl phosphate; beta-naphthyl phosphate; 4-methylumbelliferyl phosphate; and 6-bromo-2-hydroxy-3-naphtholyl-o-anisidine phosphoric acid.

Human prostatic carcinoma tissues are minced, homogenized, dialyzed and concentrated using conventional tissue extraction techniques. The crude extract is separated from extraneous water-insoluble cellular material and other proteins and then precipitated, e.g. by salting out such as with ammonium sulphate. Preferably, the partially purified isoenzymes are passed through a phosphorylated ion exchange column to increase the specific activity of the prostatic acid phosphatases. The activated enzyme is dialyzed and acid phosphatase eluted in a major and minor fraction, with the major fraction (having greater specific enzyme activity) being subjected to further purification, e.g. by gel chromatography. The molecular weight of prostatic acid phosphatase has been determined to be around 100,000 daltons. The other protein component, with a molecular weight of around 65,000 daltons, was not included in the present study. Because prostatic acid phosphatase is biochemically a glycoprotein, one can alternatively use an initial affinity chromatography, e.g. of Con A-Sepharose, for its purification. Subsequent chromatographies on a DEAE ion-exchange column and Sephadex (registered Trade Mark) G-100 gell filtrations result in a homogeneous protein preparation exhibiting acid phosphatase activity. This purified protein preparation was used for Experiments 11—24 in this study.

For the preparation of immunogens suitable for preparing diagnostic antibodies against the carcinoma-associated isoenzyme pattern in laboratory animals, conventional vaccine preparation techniques can be used. Preferably a non-antigenic adjuvant, e.g. alum, Freund's complete adjuvant, saponin, a quaternary ammonium surfactant, an alkyl amine, etc. is admixed with the purified isoenzyme proteins in a suitable immunologically acceptable, non-antigenic carrier and the resultant mixture can be sterilized, e.g. by filtration.

The vaccine can be administered parenterally following regimens already known for immunization with other proteins to stimulate the formation of immunoprecipitating antibodies, with the primary inoculation being preferably followed up by at least one additional injection one to ten weeks later. Good results have been obtained in rabbits using four booster injections at two week intervals one month after the primary immunization. The protein content per injection in rabbits, goats and other mammals can be varied, but is generally about 100 micrograms of protein per kg. of body weight. The antibodies can be collected and worked up using methods well known to those skilled in the art of immunochemistry, and provide a useful reagent for the immunological detection of prostatic acid phosphatase cancerous isoenzyme patterns in a variety of immunochemical procedures, e.g., immunoprecipitin, fluoroescent antibody, serum neutralization, etc. Such antibodies are useful as a control reagent in the diagnostic test for prostatic cancer described more particularly below. While in principle applicable to a variety of immunological tests currently in use, immunoprecipitin and fluorogenic tests represent a presently preferred embodiment and accordingly will be discussed in detail.

The simplest immunoprecipitin test involves capillary tube precipitin testing, wherein separate antibody and antigen solutions are allowed to react at a common interface in a capillary tube and a positive reaction is indicated by the formation of a precipitate at the interface. This method is relatively insensitive and inaccurate due, *inter alia,* to unavoidable diffusion of the two solutions across the interface, and furthermore the final test results cannot be preserved.

Agar gel diffusion is the simplest method which avoids these drawbacks. A solution of the antigen (or serum sample) is placed in a central well punched in a continuous agar gel and appropriate dilutions of the serum containing antibodies thereto are placed in wells concentrically surrounding the center well. A positive reaction is noted by the formation of the precipitin line between one or more of the concentric wells and the central well. This method is relatively insensitive and fairly slow, requiring 1 to 4 days to read the test results.

Radioimmunoassay (RIA), e.g. radioimmunoprecipitin tests, are extremely sensitive (by several orders of magnitude over older methods) but take several days to perform and require sophisticated equipment and highly trained personnel not always widely available.

Countercurrent electrophoresis is a widely used immunoprecipitin method which takes only about an hour to perform and which is considerably more sensitive than agar gel diffusion. Reactive components are placed in opposing wells cut into an agar gel and a small electrical current applied thereto, causing both the antigen and the antibody to migrate towards each other. While almost all immunoelectrophoresis is conducted at an alkaline pH of at least 8, it has been found that prostatic cancer isoenzymes used in the present invention become irreversibly denatured at this pH and must be run at a pH of less than 7.0, preferably 6.5 or lower. At pH 6.5, purified prostatic acid phosphatase moves towards the anode, while purified rabbit IgG moves towards the cathode. Therefore, by placing acid phosphatase in the cathodic well at the cathodic side and rabbit antisera in the anodic well, the enzyme and its antibody meet between the wells during electrophoresis. A positive reaction is indicated by the formation of a precipitate at the antigen-antibody interface. Since this test method is reasonably reliable, readily available and inexpensive, it represents the presently preferred embodiments of this aspect of the present invention.

For purposes of immunoelectrophoresis testing, the diagnostic antibody preparation of the present invention when used without purification is generally diluted with phosphate buffered saline in a volume ratio of 1:10 to 1:500, generally 1:50 to 1:250, depending on the antibody titer thereof. The limiting factor at the lower end of the range is the degree of distinction achieved in the precipitin lines, which is a function of the antibody content in the total protein present. Purified antibody preparations can of course have lower total protein concentrations, and the protein content of even the unpurified preparations can be varied to suit the particular immunochemical test to be employed, the optimal amounts being determined, e.g. by testing simple serial dilutions.

In a preferred, further aspect of the present invention, circulating prostatic acid phosphatase can now be detected at the nanogram level by immunochemical techniques, preferably by protein staining of the antibody-enzyme precipitin complex. Using the aforementioned antisera specific to prostatic acid phosphatase and coupling this with a conventional staining reagent to detect enzyme activity of the antigen-antibody complex, it is now possible to immunochemically separate the specific prostatic acid phosphatase isoenzyme pattern associated with prostate cancer from a serum sample and to detect it by the enzyme activity thereof.

The antigen-antibody complex can be stained by a number of known histochemical staining techniques, e.g. fluorescent antibody, thymolphthalein monophosphate, napthyl phosphates, phosphoric acids, etc. to increase the sensitivity of this method to 10—20 ng/ml of prostatic acid phosphatase protein, which is comparable to that obtained in radioimmunoassay techniques. Alternatively, one can use radioactive antibody for the assay, which not only provides a better quantitative value but may also further increase the sensitivity of the assay. If desired, a second enzyme, e.g. beta-galactosidase, can be coupled with purified prostatic acid phosphatase antibodies for use in an enzyme-linked immunoassay, e.g. using techniques analagous to those described by Kato et al. in J. Immunol. *116:* 1554 (1976).

In order to conserve diagnostic antibodies against the isoenzyme pattern of prostatic acid phosphatase which is associated with prostatic cancer, it is preferable to bind these antibodies onto a water-insoluble support for use in the enzyme assay. Many suitable such supports and techniques for binding proteins thereto are well known in the art and include inorganic as well as organic supports. Presently preferred are those water-insoluble supports which can be activated with a cyanogen halide, preferably cyanogen bromide, prior to the covalent bonding of the antibodies thereto, e.g. as taught by Axen et al. in U.S. Patent 3,645,852.

The presently preferred solid-phase fluorescent immunoassay technique for human prostatic acid phosphatase employs this further advantage and primarily involves the immunological specificity of prostatic acid phosphatase and the inherent fluorescent property of alpha-naphthol, the enzyme hydrolysis product of prostatic acid phosphatase. Unlike other sensitive immunoassay techniques, such as the enzyme-linked immunoassay or the double antibody radioimmunoassay, this technique does not require the application of a second enzyme or antibody. Further, the quantitation of prostatic acid phosphatase in this assay is based upon the catalytic activity of the enzyme and therefore differs from that of radioimmunoassay, which measures the mass of the enzyme protein. The specificity of this assay is provided by the antiprostatic acid phosphatase, raised against the purified enzyme from the prostate, which specifically binds the prostatic acid phosphatase; the assay is further characterized by the fluorescence of enzyme hydrolysis product, which permits quantitation with a very sensitive spectrophotofluorometric technique. It therefore combines an immunological and biochemcial as well as a chemical approach to enzyme quantitation.

The sensitivity of this solid-phase immunofluorescent assay for prostatic acid phosphates is 60 pg/ml of serum under the experimental protocol described. If a greater sensitivity is needed, it can be accomplished by increasing the volume of specimen assayed. The sensitivities of the counter immunoelectrophoresis and the radioimmunoassay techniques are 20 and 10 ng/ml, respectively. Immunofluoroassay, therefore, provides a more sensitive tool for serum prostatic acid phosphatase determinations. This procedure is reliable, as supported by reproducibility studies of within-assay and between-assay. The solid-phase IgG (anti-prostatic acid phosphatase)-Sepharose can be reused. Furthermore, this assay does not require the use of an isotope. The standard curve of this assay extends from 60 pg to 912 ng per ml of specimen, so that it covers a much broader range than the radioimmunoassay.

Without further elaboration, it is believed that one skilled in the art can, using the preceding description, utilize the present invention to its fullest extent. The following preferred specific embodiments are, therefore, to be construed as merely illustrative and not limitative of the remainder of the disclosure in any way whatsoever. All temperatures are set forth uncorrected in degrees Celsius; unless otherwise indicated, all pressures are ambient and all parts and percentages are by weight.

Example 1

Extraction of Crude Material

Human prostatic tissues and other human tissues (kidney, lung, liver etc.) were collected during autopsy or surgery. All tissues were used immediately after collection or stored immediately after collection at −75°C. until used. Normal male and female sera were donated by volunteers. Sera of patients with histologically proven prostatic carcinoma and the sera of patients bearing other cancers were obtained from the Roswell Park Memorial Institute and the National Prostatic Cancer Project chemotherapy participating institutes; Mount Sinai School of Medicine provided sera from patients with Gaucher's disease. The clinical stage of patients with prostate cancer was determined by the system of Whitemore described in Cancer *16:* 1119 (1963) and summarized as follows: Stage A — tumors confined to the prostate and not palpable; Stage B — tumors confined to the prostate but palpable, no metastasis; Stage C — locally invasive tumors, induration extending beyond the capsule of the prostate; and Stage D — distant metastases, e.g. bones or soft tissue.

The tissues were weighed, minced and mixed with 0.02M sodium acetate buffer, pH 5.2 containing 0.1% of Tween (registered Trade Mark) 80 (3 ml/g tissue). Homogenization was carried out with an Omni-mixer. Tissues in the homogenizer were subjected to 5 minutes blending, three times, at a blade speed of 25,000 rpm with intermittent cooling times of 3 minutes. The homogenates were stirred overnight, then centrifuged at 10,000 × g for 20 minutes. The supernatant was dialyzed overnight against 0.02M sodium acetate buffer, pH 5.2. The dialyzed supernatant was concentrated to desired volume in a concentrator fitted with a Diaflo PM 10 ultra-filter under nitrogen pressure. The extracts of other tissues were dialyzed against water for 2 days with 3 changes of water at 12 hour intervals. This entire operation and all other experiments were carried out at 4°C. The dialyzed materials were then concentrated to about 15—30 mg/ml protein.

Example 2

Initial Purification of Acid Phosphatase

The crude extract which was obtained in Example 1 from 130 g of tissue was passed through 0.5 × 6 cm column of agarose: 5'-(*p*-nitrophenyl phosphate) uridine-2'-(3') phosphate (commercially available from Miles Laboratories, Elkhart, Indiana) to remove ribonuclease and other proteins. Ammonium sulphate was added to the effluent (containing acid phosphatase) to 75% saturation. After 12 hours, the mixture was centrifuged and the pellet dissolved in a minimum amount of 0.01M phosphate buffer, pH 6.0, and dialyzed overnight against the same buffer.

Example 3

Specific Activity Enhancement of Acid Phophatase

The solution from Example 2 was passed through 2.5 × 46 cm phosphocellulose column equilibrated with 0.02M phosphate buffer, pH 6.0. Acid phosphatase was not adsorbed on this column, but the specific activity of the enzyme (enzyme activity per unit weight of protein) was increased approximately 60—100% by this step. The resultant effluent was dialyzed against 0.02M phosphate buffer, pH 7.0 and applied onto a 2.5 × 35 cm DEAE—Sephadex A50 column pre-equilibrated with the same buffer. The column was first washed with 300 ml of buffer, followed by a convex gradient of 0 to 0.5M NaCl in 0.02M phosphate buffer pH 7.0 to 6.0 with 400 ml of 0.02M phosphate buffer pH 7.0 in the reservoir and 200 ml of 0.02M phosphate buffer, pH 6.0 containing 0.5 M NaCl, in the mixer. Acid phosphatase was eluted out in one major and one minor fraction. Only the major fraction with a greater specific enzyme activity was subjected to further purification.

Example 4

Purification of Activated Acid Phosphatase

Eluates containing acid phosphatase were pooled and dialyzed overnight against 0.02M phosphate buffer, pH 7.0. The dialyzed enzyme solution was concentrated by adsorption on a small column (1.8 × 6 cm) of DEAE—Sephadex A50, followed by elution with 0.02M phosphate buffer, pH 6.0. A concentrated solution (20 ml) was applied to a Sephadex G-200 column (3.5 × 106 cm) and the column was eluted with 0.02M phosphate buffer, pH 7.0. Fractions containing the final purified acid phosphatase were pooled and used for further experiments.

## Example 5

Isoelectric Focusing

Isoelectric focusing was carried out at 4°C. using Shandon Southern analytic polyacrylamide gel electrophoresis apparatus. Polyacrylamide gels (5 x 70 mm) were prepared according to procedures described in the instruction sheet from the Bio-Rad Laboratories. Phosphoric acid (0.02 M) was used as the anolyte and NaOH (0.01 M) as the catholyte. Samples were mixed with an equal volume of 50% sucrose solution and 50 mu l of sample sucrose solution was applied on top of the gels (anode side). A constant voltage of 120 volts was applied for 20 hours. After electrophoresis gels were stained for acid phosphatase activity using 0.1% alpha-naphthyl phosphate — 0.1% fast Garnet GBC salt in 0.1 M ammonium acetate buffer at pH 5.0.

## Example 6

Homogenity of Purified Enzyme

Upon polyacrylamide gel electrophoresis, this enzyme preparation was shown to be homogeneous, with a single enzyme activity band overlapping with the protein band. Diffuse bands were observed in the experiment, probably due to the fact that prostatic acid phosphatase is biochemically a glycoprotein. The protein and acid phosphatase activity profiles of the final. step of purification on Sephadex G-200 were determined. A symmetrical protein peak (at 280 nm), exhibiting acid phosphatase activity (at 400 nm), was obtained. With this modified isolation procedure, a 150-fold purification of enzyme was achieved, which was 50% higher than with the procedure previously described in Cancer Chemotherapy Reports *59:* 97 (1975).

## Example 7

Simplified Purification of Acid Phospatase From Prostate Tissues

102.5 g of prostatic tissue was homogenized with 308 ml of 0.01% EDTA-0.1% PBS, pH 6.8. The extract was centrifuged at 28,000 x g for 30 minutes at 4°C. and the supernatant (350 ml) was precipitated with 50—70% ammonium sulfate. The precipitate was dissolved in the starting buffer solution (1 mM each of CaCl, $MnCl_2$ and $MgCl_2$ in 0.1M NaCl, NaOH acetate) and dialyzed overnight against the starting buffer. The solution was centrifuged at 28,000 g for 3 minutes at 4°C. The supernatant (31 ml) was then applied onto a Concanavalin A-Sepharose column (2 x 40 cm) and incubated overnight at 4°C. The unbound proteins were eluted with the starting buffer solution and the bound proteins (primarily acid phosphatase) were eluted with 500 ml of 0.1 M—0.5 M methyl-alpha-D-manno-pyranoside in starting buffer. The fractions containing acid phosphatase were concentrated to 6.8 ml with an Amicon PM-30 membrane, and this concentration solution was subjected to a DEAE cellulose column (2 x 46 cm) and eluted with a linear gradient 500 ml of 0.2M sodium phosphate pH 7.0—500 ml of 0.02M sodium phosphate pH 6.0. The fractions containing acid phosphatase were again concentrated to 3.5 ml with an Amicon PM-30 membrane, applied onto a Sephadex G-100 column (2.5 x 96 cm) and eluted with 0.01M citrate buffer, pH 6.0. A homogeneous protein exhibiting acid phosphatase was thus obtained. The relative enzyme activity and purification of acid phosphatase at each step of this procedure is shown in the attached table.

| Step | Vol ml | Enzymic Activity (Unit) | Protein Conc. (mg/ml) | Total Protein (mgs) | Total Enzymic Activity (Unit) | Specific Activity | Relative Activity (fold) |
|---|---|---|---|---|---|---|---|
| Prostate Tissue | 350 | 393 | 23.5 | 8225 | 137550 | 16.7 | 1 |
| $(NH_4)_2SO_4$ Precipitate | 31 | 1861 | 9.5 | 294.5 | 57691 | 195.8 | 11.7 |
| Con A Column | 6.8 | 3933 | 19.0 | 129.2 | 26744 | 207 | 12.4 |
| DEAE Column | 3.5 | 8390 | 11.5 | 40.3 | 29365 | 729 | 43.7 |
| G-100 Column | 2.7 | 4391 | 3.28 | 8.8 | 11855 | 1338.7 | 80.2 |

## Example 8

### Preparation of Antisera

Purified prostatic acid phosphatase obtained from Example 4 was emulsified with an equal volume of complete Freund's adjuvant, and 1 ml of emulsified mixture containing 100 micrograms of protein was injected subcutaneously among ten sites on the back of each of eight female rabbits. Similar preparations were injected into three goats using 3 mg of protein per goat. A booster injection using the same dosage as the primary immunization was given one month later and repeated three times at two week intervals. Collection of antisera started 10 to 14 days after the last injection. Antisera were obtained by drawing the blood directly from the ear vein. The blood was allowed to clot at room temperature and then centrifuged.

The antisera are specific to prostatic acid phosphatase. By double diffusion and countercurrent immunoelectrophoresis techniques, using crude acid phosphatase preparations from normal kidney, bladder, bone, liver, spleen, brain and intestine at concentrations of 3 mg protein/ml and 20—500 I.U. (International Units, the number of micromoles of substrate converted per minute per liter of enzyme solution) enzyme activity, no reaction was shown with the antisera. Furthermore, no precipitate formation was observed when the antisera was reacted with normal male or female human serum samples. Antisera absorbed by normal female serum and extracts of these various tissues (at 1 mg/ml each) continued to react with prostatic acid phosphatase preparations and with sera from patients having prostate cancer. The unabsorbed antisera did not cross-react with acid phosphatase of plant (potato) origin. The specificity of the antisera to acid phosphatase was further confirmed by countercurrent immunoelectrophoresis with the reagent antisera replaced by normal rabbit antisera, wherein no precipitin lines were observed.

## Example 9

### Countercurrent Immunoelectrophoresis

Various conditions for the electrophoresis were examined. These include the concentration of agar, pH, temperature, current, length of time of electrophoresis, dilution of antisera, size of well and distance between the wells. Detection of the enzyme-antibody precipitin line could not be achieved by regular protein stains (Coomassie solution or Amido Black), as the concentration of serum prostatic acid phosphatase was too minute to be visualized. The application of enzyme activity staining with 0.1% alpha-naphthyl phosphate and 0.1% fast Garnet GBC salt reagent in 0.1M ammonium acetate buffer at pH 5.0 analagous to the Veronal acetate buffer technique described by Barkat in J. Histochem. and Cytochem. *9:* 542 (1961) greatly increased the sensitivity of the technique. Many other staining reagents were also successfully employed as shown in the following Table and it appears that any of the known acid phosphatase susbtrates can be used. The present optimal staining conditions for enzyme activity are reported below.

| Substrate | Staining Agents |
| --- | --- |
| 1. Sodium Glycerophosphate | Ammonium Sulfide |
| 2. Naphthol-AS Phosphate | Fast Violet B Salt |
| 3. Alpha-Naphthyl Phosphate | Fast Blue RR |
| 4. Alpha-Naphthyl Phosphate | 5-Chloro-o-toluidine-diazonium salt |
| 5. Naphthol-AS—MX Phosphoric Acid | Fast Garnet GBC Salt |
| 6. Naphthol-AS—BI Phosphoric Acid | Fast Garnet GBC Salt |
| 7. Thymolphthalein Monophosphate | Fast Garnet GBC Salt |

Countercurrent immunoelectrophoresis was performed on plastic mylar sheets [Mylar (registered Trade Mark) 6509 13 x 18 commercially available from Bio-Ware, Inc., Wichita, Kansas] covered with 35 ml of 0.75% (Sigma Chemical Co., St. Louis, Missouri) agarose in 0.05M phosphate buffer at pH 6.5. The same buffer was used in the electrode vessels. Parallel rows of wells, 4 mm in diameter, were cut 5 mm apart in the agarose. Ten microliters of serum was placed in each cathodic well and an equal volume of diluted antisera added to each anodic well. (The proper antibody concentration was determined by electrophoresing serially diluted antibody against 4 I.U. or 0.2 ng of purified acid phosphatase and choosing the lowest concentration of antibody giving a suitable reaction). The agarose sheet was then placed in the electrophoresis cell, with the ends of the sheet dipping into the buffer in the electrode vessels. A plate of this size can accommodate as many as three double rows containing eighteen paired wells in each row. Thus, 54 samples can be analyzed on each plate and the two plates can be run simultaneously. The whole procedure can be performed with ease in 6—8 hours.

A constant current of 3.2 mamp/cm was applied for 2 hrs. at 4°C. The plate was then stained for 1 hour at 37 degrees C. in 0.1% alpha-naphthyl phosphate and 0.1% fast Garnet GBC salt in 0.1M ammonium acetate buffer at pH 5.0. The plate was rinsed in distilled water and the reaction evaluated. A positive result was reported when the serum specimen contained a detectable amount of prostatic acid phosphatase by this method. After evaluation, the plates were air dried at room temperature and stored for future reference.

The sensitivity of the assay varied from run to run, depending upon the antisera used. The lowest detectable activity of serum prostatic acid phosphatase using 10 microliter specimens ranged from 0.2—0.4 I.U. or 10—20 ng/ml of prostatic acid phosphatase protein.

Example 10

Clinical Diagnostic Results

Using the countercurrent immunoelectrophoresis technique of Example 9, sera from patients with prostatic cancer were examined. In one patient with proven stage A disease, no serum prostatic acid phosphatase could be detected by this method. However, of 20 patients with proven stage B, (6/20 or 30%) gave positive results. As the disease became more severe, the percentage of positives increased; 55% (27/49) of patients with proven stage C and 80% (98/125) in proven Stage D were detectable. None of 19 patients with benign prostatic hypertrophy gave any positive results.

In order to determine the rate of possible false positives, serum specimens from 107 normal healthy volunteers and 50 normal age-matched older men were similarly examined; all had negative results. In addition, 87 patients with other carcinomas of the colon, lung, stomach, pancreas and kidney were tested, including seven female patients with metastasized (to bone) breast cancer. Only one of these 87 patients gave a positive result; this patient turned out to have both a primary lung adenocarcinoma and prostate cancer.

As previously noted, it has been reported that a significant number of patients with Gaucher's disease have an elevated serum acid phosphatase activity. Therefore, sera from 12 patients with Gaucher's disease were tested; all gave negative results.

Example 11

Alternate Purification of Prostatic Acid Phosphatase

The supernatant containing the crude acid phosphatase preparation from 102 g of prostatic tissue was brought to 40% saturation of ammonium sulfate with mixing, settled for 3 hr at 0° and then centrifuged at 13,000 rpm for 30 min. The supernatant was adjusted to 75% saturation of ammonium sulfate, mixed, kept at 0° for 3 hr, and centrifuged at 13,000 rpm for 30 min at 4°. The precipitate was dissolved in a minimum amount of starting buffer, pH 5.0 (1 mM each for $CaCl_2$, $MgCl_2$, and 0.1 M each for NaCl and $CH_3COONa$), dialyzed overnight against the same buffer and then centrifuged at 13,000 rpm for 30 min at 4°. The supernatant (30 ml) was applied to a Con A-Sepharose column (2 × 40 cm) and incubated for 24 hr at 4°. The proteins not bound to the column were eluted with starting buffer (Peaks I, II). The acid phosphatase and other glycoproteins, bound to Con A, were eluted with a linear concentration gradient of 0.1 to 0.5 M alpha-methyl-D-mannopyranoside with the use of 500 ml of the above starting buffer in each reservoir (Peak III).

The eluate containing acid phosphatase (peak 111) was dialyzed against 0.02 m phosphate buffer, pH 7.0, for 18 hr and concentrated to 3 to 4 ml with a Diaflo PM-10 membrane ultrafilter. This solution was applied to a DEAE-cellulose column (2×46 cm), equilibrated with 0.02 M phosphate buffer, pH 7.0, and eluted with a linear gradient of 0 to 0.5 M NaCl in 0.02 M phosphate buffer, pH 7.0 to 6.0. Three fractions exhibiting acid phosphatase activity were obtained (Fractions I, II and III). The Fraction (Tubes 79 to 94) that contained the major protein peak was further purified by being passed through a Sephadex 6—100 column (2.5 × 96 cm) and eluted with 0.01 n citrate buffer, pH 6.0. Acid phosphatase was separated into 1 major and 1 minor fraction. The first (major) fraction, containing the acid phosphatase with a molecular weight of 100,000, was rechromatographed on Sephadex G—100 and a homogeneous preparation was obtained. This purified acid phosphatase preparation was used for further experiments.

Example 12

Disc Electrophoresis on Polyacrylamide Gel

Disc electrophoresis was performed by the technique described by Davis in Ann. N. Y. Acad. Sci. *121:* 404—427 (1976) in a standard 7.5% polyacrylamide gel. Twenty-five micrograms of purified enzyme, containing 25% of sucrose to increase the density of a tested solution, were applied to the top of a 5 × 70 mm polyacrylamide gel column. Electrophoresis was carried out in 0.05 Tris-HCl buffer, pH 8.3, at 4°, with a constant current of 5 ma/tube for 1 hr. After electrophoresis, the gels were pushed out of the glass tubes and the protein was detected by staining (30 min) with 0.1% Coomassie blue R-250. The gel was destained with 5 to 10% methanol by volume. The enzymatic activity of acid phosphatase was detected by staining with 0.1% alpha-naphthyl phosphate-0.1%. Fast Garnet GBC salt in 0.1 M ammonium acetate buffer, pH 5.0 and the stains in the gels were then scanned.

## Example 13
### Assay of Enzyme Activity

Acid phosphatase activity in the chromatographic fractions was determined by the method of Babson and Phillips described in Clin. Chim. Acta *13:* 264—265 (1966). This method uses alpha-naphthyl phosphate as the substrate; the hydrolyzed product, alpha-naphthol, forms a stable colored complex with Fast Red Salt B in an alkaline condition. The absorbance was measured at 588 nm. One IU of acid phosphatase activity is defined as the amount of enzyme in 1 liter of sample that willl hydrolyze the substrate at a rate of 1 micro mol/min.

Determination of Protein Concentration: The method of Lowry et al. described in J. Biol. Chem. *193:* 265—275 (1951) was used, with bovine serum albumin used as the standard.

Preparation of Antisera: The anti-prostatic acid phosphatase serum was raised by injecting the purified enzyme and a complete Freund's adjuvant into female rabbits as previously described in Investigative Urology *15:* 319—323 (1978).

## Example 14
### Purification of Anti-Prostatic Acid Phosphatase IgG

The method described by Harboe and Ingred in Scand. J. Immunol. Suppl. *1:* 161—164 (1973) was used. Briefly, rabbit anti-prostatic acid phosphatase serum (20 ml) was added to 10 ml saturated ammonium sulfate and thoroughly mixed. The mixture was kept in 0° for 3 hr and centrifuged at 2,000 rpm for 30 min; the precipitate was dissolved in sodium phosphate buffer, pH 6.3, and then dialyzed against the same buffer for 24 hr at 4°. The dialyzed solution was applied to a DEAE-cellulose column (1 x 40 cm) and eluted with 0.0175 M sodium phosphate buffer, pH 4.3. The IgG antibody eluted at the first protein peak.

Conjugation of purified antiprostatic acid phosphatase IgG sepharose 4B was carried out according to the manufacturer's recommended procedure (Pharmacia Fine Chemicals of Uppsala, Sweden). The CNBr activated sepharose 4B (1g) was washed and reswelled on a centered glass filter with one mM HCl. The purified antiprostatic acid phosphatase IgG (5 to 10 mg protein per ml gel) was dissolved in 0.1 m $NaHCO_3$ buffer pH 8.5 containing 0.5 m NaCl and mixed with CNBr activated sepharose 4B gel suspension, end over end, for 2 hours at room temperature. After incubation, the excess IgG was removed by means of the coupling buffer. The remaining active groups on Sepharose 4B were blocked by adding 1 M monoethanolamine solution, pH 9.0 (5 ml), with gentle mixing for 2 hr at room temperature. Finally, excess blocking reagent was removed by washing first with acetate buffer (0.1 M, pH 4.0) containing 0.5 M NaCl and then with the coupling buffer. This IgG-Sepharose 4B conjugate was further washed with PBS and stored at 4° until used. This procedure resulted in coupling of about 98% of the IgG to the CNBr-activated Sepharose 4B, as determined by measuring immunoglobulin concentration before and after the coupling reaction by spectrophotometry at 280 nm.

## Example 15
### Reactivity of IgG-Sepharose-bound Acid Phosphatase

The reactivity of IgG-Sepharose-bound acid phosphatase was studied as follows. Purified acid phosphatase (0.2 ml in PBS) was incubated with IgG-Sepharose (200 microliters) at room temperature for 2 hr; the resulting Sepharose IgG-acid phosphatase was washed with PBS 3 times. The Sepharose-IgG-acid phosphatase was kept at 4°. Another aliquot, 0.2 ml of acid phosphatase, was also left at room temperature for 2 hr and then kept at 4°. Aliquots of 20 microliters each were taken at 8 hr intervals and assayed for enzyme activity. Twenty microliters of specimen were mixed with 1 ml of substrate (see below) and incubated at 37° for 15 min. The reaction was stopped by adding 0.1 M NaOH (2.5 ml), and the hydrolyzed fluorogenic product, alpha-naphthol, was measured with a spectrophotofluorometer.

## Example 16
### Solid-Phase Fluorescent Immunoassay

Patient's serum (50 microliters) or prostatic acid phosphatase was incubated with IgG-Sepharose 4B (50 microliters) in polystyrene tubes (8 x 75 mm) in PBS for 2 hr at room temperature and then overnight at 4°. The prostatic acid phosphatase was bound to the IgG on the Sepharose 4B. After centrifugation and washing of the precipitate 3 times with PBS, 1.0 ml of 3 mM alpha-naphthyl phosphate (substrate) in 0.2 M citrate buffer, pH 5.6, was added to the acid phosphatase-IgG-Sepharose 4B, and this was incubated for 1 hr at 37°. The supernatant (0.8 ml) was transferred to a new tube containing 2.5 ml of 0.1 M NaOH. The enzyme activity was determined by an Aminco spectrophotofluorometer, with excitation at 340 nm and emission at 465 nm. A standard curve was established with various concentrations of purified acid phosphatase under identical experimental conditions, and the quantity of prostatic acid phosphatase in patients' serum was determined from the standard curve.

## Example 17

Recycling of IgG-Sepharose 4B

After the enzyme activity was measured, the prostatic acid phosphatase which bound to the IgG-Sepharose 4B was dissociated by the use of 5 M guanidine-HCl, pH 8.5, at room temperature for 30 min. The guanidine-HCl and prostatic acid phosphatase were removed by washing with PBS overnight. The dissociated and reactivated IgG-Sepharose can be reused for at least 2 more experimental runs.

## Example 18

Homogeneity and Specificity

The human prostatic acid phosphatase was purified to homogeneity by the procedure described above. After a series of chromatographies on a Con A affinity column, a DEAE ion-exchange column and Sephadex G-100 gel filtration, a symmetrical protein peak exhibiting acid phosphatase activity was obtained. The homogeneity of the purified protein was confirmed by disc polyacrylamide gel electrophoresis, which demonstrated a single protein band superimposed on the enzyme activity band. The molecular weight of the prostatic acid phosphatase was estimated to be 100,000 by gel filtration by the use of Sephadex G-200. With this procedure, an 85-fold purification and 38% recovery of the activity was achieved.

The anti-prostatic acid phosphatase serum, when reacted with prostatic acid phosphatase, gave a single precipitin line on gel diffusion when stained for both protein and acid phosphatase activity. Similarly, after immunoelectrophoresis, only a single arc was observed when stained for protein and enzyme activity against purified prostatic acid phosphatase or crude extract of prostatic tissues. The antiserum did not show any immunological cross reactivity with the acid phosphatases extracted from other human tissues such as liver, kidney, intestine, lung, etc.

The anti-prostatic acid phosphatase IgG fraction was purified from the antiserum and conjugated to CNBr-activated Sepharose 4B. The IgG (anti-prostatic acid phosphatase)-Sepharose 4B was then used to specifically bind the prostatic acid phosphatase.

## Example 19

Reactivity of IgG Bound Acid Phosphatase

The enzyme activity of acid phosphatase was studied with and without binding to IgG (anti-prostatic acid phosphatase)-Sepharose 4B. The acid phosphatase which bound to IgG-Sepharose exhibited enzyme activity; furthermore, no loss of the enzyme activity was demonstrated for 48 hr whereas the "free" acid phosphatase (not bound to IgG-Sepharose) lost about 64% of its enzyme activity during the same 48 hr period and 87% of activity after 96 hr. These results indicate that the IgG-Sepharose-bound acid phosphatase retained its catalytic reactivity for at least 48 hr under these experimental conditions.

## Example 20

Minimum Amount of Solid-phase (IgG-Sepharose) Required

In order to determine the minimum amount of IgG (anti-prostatic acid phosphatase)-Sepharose 4B necessary for the assay, various concentrations of acid phosphatase were incubated with a constant amount of IgG-Sepharose for 2 hr at room temperature and overnight at 4°. The enzymatic activity of the acid phosphatase bound to IgG antibody was determined by the fluorometric technique described above. Results indicated that 50 microliters of IgG (antiprostatic aid phosphatase)-Sepharose (containing 0.28 mg of antiprostatic acid phosphatase-IgG) would bind 45.6 ng of prostatic acid phosphatase. Therefore, a minimum amount of IgG (anti-prostatic acid phosphatase)-Sepharose (0.28 mg of IgG) was used in this solid-phase fluorescent immunoassay in order to measure the serum prostatic acid phosphatase concentration up to 912 ng/ml without diluting the serum samples.

## Example 21

Incubation Conditions for Fluorescent Immunoassay

In order to determine the optimal incubation conditions for the solid-phase fluorescent immunoassay, prostatic acid phosphatase was incubated with the IgG-Sepharose 4B under different temperatures and at various time intervals. The enzyme activity was also assayed at different incubation times with the alphanaphthyl phosphate susbtrate. The results revealed that incubation first at room temperature for 2 hr and then overnight at 4° afforded the best binding between prostatic acid phosphatase and IgG-Sepharose. The measurement of enzyme activity was best determined at 37° for 1 hr.

## Example 22

Sensitivity and Reproducibility of Fluorescent Immunoassay

The sensitivity of this immunoassay was verified by performing various triplicate determinations of prostatic acid phosphatase, ranging from 45.6 ng to 3 pg in 50 microliters of specimen. The prostatic acid phosphatase at 3 pg/50 microliters could be detected by this immunofluorometric assay. This assay procedure was found to be reproducible, as a within-assay standard deviation of 6 pg/50 microliters (10 determinations) and a between-assay standard deviation of 9 pg/50 microliters (3 assays, 3 determinations in each assay) were obtained from a sample of 280 pg/50 microliters, representing a coefficient of variation of less than 4%.

Example 23

Recycling of Solid Phase Reagents

The IgG (anti-prostatic acid phosphatase)-Sepharose, after the dissociation of acid phosphatase from the prostatic acid phosphatase-IgG-Sepharose, can be reused for the assay. Although a slight decrease of sensitivity occurred, it could be recycled at least three times without losing any appreciable binding to prostatic acid phosphatase.

Example 24

Additional Clinical Diagnostic Results

The results of an inital application of this newly developed immunofluoroassay in testing sera from patients with prostate cancer and other tumors were determined. The determination of serum prostatic acid phosphatase by this assay from a group of 30 apparently healthy male volunteers resulted in a mean of 5.619 ng/ml with a standard deviation of 2.110. A normal range of 1.399 to 9.839 ng/ml was thus determined. The serum prostatic acid phosphatase levels from 24 patients with all stages of prostate cancer with studied in this preliminary report. The enzyme was found to be elevated in 4 untreated patients with Stage A disease, in 2 of 5 with Stage B and in 7 of 11 with Stage C who were receiving standard estrogen therapy and/or radiation therapy, and in all 4 patients with Stage D disease receiving chemotherapy. On the other hand, the serum prostatic acid phosphatase levels were in the normal range in all 16 patients with other advanced tumors, such as cancer of the lung, breast, colon, rectum, stomach or pancreas. These 16 specimens were randomly chosen from serum samples that had exhibited a highly elevated level of carcinoembryonic antigen (all had a value of greater than 15 ng/ml).

The preceding examples can be repeated with similar success by substituting the generically or specifically described reactants and/or operating conditions of this invention for those specifically used in the examples.

Industrial Applicability

As can be seen from the present specification, particularly Examples 10 and 24 thereof, the present invention is particularly applicable in diagnosing the presence of antigenic isoenzyme patterns associated with prostatic cancer, even at relatively early stages thereof.

**Claims**

1. An in vitro composition comprising an immunochemically effective amount of antibodies against the human prostatic acid phosphatase isoenzyme pattern of the isoenzyme forms having an isoelectric point range (pI) of 4.1—5.5 which are associated with prostatic cancer, said composition being substantially free of antibodies against the normal human serum prostatic acid phosphatase isoenzyme forms, against acid phosphatases extracted from other body tissues and against antigens associated with other carcinomas or Gaucher's disease.

2. A composition according to Claim 1, wherein said isoelectric point range is pI 4.5—5.0.

3. A composition according to Claim 1, wherein said antibodies are immunoprecipitating antibodies.

4. A composition according to Claim 3, wherein said antibodies are labeled for radioimmunoassay.

5. A composition according to Claim 1, wherein said antibodies are covalently bonded to a water-insoluble support.

6. A method for diagnosing elevated serum acid phosphatase levels in the characteristic isoenzyme pattern of the isoenzyme forms associated with the early stages of prostatic cancer, which comprises forming an immunoprecipitin complex between an antigen consisting essentially of said serum prostatic acid phosphatase isoenzyme pattern and immunoprecipitating antibodies specific thereto which are substantially free of immunoprecipitating antibodies against the normal human serum prostatic acid phosphatase isoenzyme forms, against acid phosphatases extracted from other body tissues and against antigens associated with other carcinomas or Gaucher's disease, and detecting the presence of said complex.

7. A method according to Claim 6, wherein the complex is formed by countercurrent immunoelectrophoresis at a pH of less than 7.0.

8. A method according to Claim 7, wherein the presence of said complex is detected by measuring the acid phosphatase enzyme activity thereof.

9. A method according to Claim 8, wherein said enzyme activity is measured by staining with a colormetric substrate for said acid phosphatase.

10. A method according to Claim 9, wherein said substrate comprises alpha-naphthyl phosphate.

11. A method according to Claim 6, wherein the complex is formed by immunologically reacting said acid phosphatase with anti-prostatic acid phosphatase antibody covalently bound to a water-insoluble support.

12. A method according to Claim 11, wherein the presence of said complex is detected by measuring the acid phosphatase enzyme activity thereof.

13 A method according to Claim 12, wherein said enzyme activity is measured by staining with a colormetric substrate for said acid phosphatase.

14. A method according to Claim 13, wherein said substrate produces a fluorogenic hydrolysis product upon reaction with said enzyme and the presence of said hydrolysis product is measured spectrophotometrically.

15. A method according to Claim 14, wherein said substrate comprises alpha-naphthyl phosphate.

## Revendications

1. Composition pour un emploi in vitro, comprenant une quantité immunochimique d'anti-corps contre le type isoenzyme de phosphatase acide de la prostate humaine des formes isoenzymatiques ayant un point isoélectrique (pI) de 4,1 à 5,5, dont la présence est liée au cancer de la prostate, cette composition étant essentiellement dépourvue d'anti-corps contre les formes isoenzymatiques de phosphatase acide prostatique du sérum humain normal, contre les phosphatases acides extraites d'autres tissus corporels ainsi que contre les antigènes dont la présence est liée à d'autres carcinomes ou à la maladie de Gaucher.

2. Composition selon la revendication 1, dans laquelle le point isoélectrique est compris entre 4,5 et 5,0.

3. Composition selon la revendication 1 ou 2, dans laquelle les anti-corps sont des anti-corps immunoprécipitants.

4. Composition selon la revendication 3, dans laquelle les anti-corps sont marqués pour un dosage radio-immunologique.

5. Composition selon la revendication 1, dans laquelle les anti-corps sont liés par liaison covalente à un support insoluble dans l'eau.

6. Méthode pour diagnostiquer des taux élevés de phosphatase acide sérique dans le type isoenzymatique caractéristique des formes isoenzymatiques, dont la présence est liée aux premiers stades du cancer de la prostate, méthode selon laquelle on forme un complexe d'immuno-précipitine entre un antigène consistant essentiellement en ce type de phosphatase acide prostatique sérique et des anti-corps immunoprécipitants spécifiques à son égard, qui sont pratiquement dépourvus d'anti-corps immuno-précipitants contre les formes isoenzymatiques de phosphatase acide prostatique de sérum humain normal, contre les phosphatases acides extraites d'autres tissus corporels ainsi que contre les antigènes dont la présence est liée à d'autres carcinomes ou à la maladie de Gaucher, et on détermine la présence de ce complexe.

7. Méthode selon la revendication 6, dans laquelle on forme le complexe par immuno-électrophorèse à contre-courant à un pH inférieur à 7.0.

8. Méthode selon la revendication 7, dans laquelle on détermine la présence du complexe en mesurant son activité enzymatique en ce qui concerne la phosphatase acide.

9. Méthode selon la revendication 8, dans laquelle on mesure l'activité enzymatique par coloration avec un substrat colorimétrique pour la phosphatase acide.

10. Méthode selon la revendication 9, dans laquelle le substrat comprend du phosphate d'$\alpha$-naphtyle.

11. Méthode selon la revendication 6, dans laquelle le complexe est formé par réaction immunologique de la phosphatase acide avec un anti-corps anti-phosphatase acide prostatique lié par liaison covalente à un support insoluble dans l'eau.

12. Méthode selon la revendication 11, dans laquelle la présence du complexe est déterminée par mesure de son activité enzymatique en ce qui concerne la phosphatase acide.

13. Méthode selon la revendication 12, dans laquelle l'activité enzymatique est mesurée par coloration avec un substrat colorimétrique pour la phosphatase acide.

14. Méthode selon la revendication 13, dans laquelle le substrat forme un produit d'hydrolyse fluorogène par réaction avec l'enzyme, et la présence de ce produit d'hydrolyse est déterminée par spectrophotométrie.

15. Méthode selon la revendication 14, dans laquelle le substrat comprend du phosphate d'$\alpha$-naphtyle.

## Patentansprüche

1. Eine in vitro (Reagenzglasversuch) -Zusammensetzung, enthaltend eine wirksame Menge von Antikörpern gegen das Säurephosphataseisoenzymmuster der menschlichen Prostata derjenigen Isoenzymformen, deren Bereich für den isolektrischen Punkt (pI) bei 4,1—5,5 liegt, und die mit Prostatakrebs

**0 018 967**

assoziiert sind, dadurch gekennzeichnet, daß diese Zusammensetzung im wesentlichen frei ist von Antikörpern gegen die normalen Serum-Säurephosphataseisoenzymformen der menschlichen Prostata, gegen Säurephosphatasen, die aus anderen Körpergeweben extrahiert worden sind, und gegen Antigene, die mit anderen Karzinomen oder mit Gaucher's Krankheit assoziiert sind.

2. Zusammensetzung nach Anspruch 1, dadurch gekennzeichnet, daß dieser Bereich für den isoelektrischen Punkt bei 4,5—5,0 liegt.

3. Zusammensetzung nach Anspruch 1, dadurch gekennzeichnet, daß diese Antikörper immunopräzipitierende Antikörper sind.

4. Zusammensetzung nach Anspruch 3, dadurch gekennzeichnet, daß diese Antikörper für Radioimmunotests markiert sind.

5. Zusammensetzung nach Anspruch 1, dadurch gekennzeichnet, daß diese Antikörper an einen wasserunlöslichen Träger kovalent gebunde sind.

6. Verfahren zum Diagnostizieren von erhöhten Serumsäurephosphatasespiegeln im charakteristischen Isoenzymmuster der Isoenzymformen, die mit den frühen Stadien von Prostatakrebs assoziiert sind, dadurch gekennzeichnet, daß man einen Immunopräzipitinkomplex bildet zwischen einem Antigen, das im wesentlichen aus diesem Serum-Säurephosphataseisoenzymmuster aus der Prostata besteht, und hierfür spezifischen immunopräzipitierenden Antikörpern, welche im wesentlichen frei sind von immunopräzipitierenden Antikörpern gegen die normalen Serumsäurephosphataseisoenzymformen der meschlichen Prostata, gegen aus anderem Körpergewebe extrahierte Säurephosphatasen, und gegen mit anderen Karzinomen assoziierte oder mit Gaucher's Krankheit assoziierte Antigene, und daß man das Vorhandensein dieses Komplexes festellt.

7. Verfahren nach Anspruch 6, dadurch gekennzeichnet, daß der Komplex gebildet wird durch Gegenstrom-Immunoelektrophorese bei einem pH-Wert unter 7.0.

8. Verfahren nach Anspruch 7, dadurch gekennzeichnet, daß das Vorhandensein dieses Komplexes festgestellt wird durch Messung seiner Säurephosphataseenzymaktivität.

9. Verfahren nach Anspruch 8, dadurch gekennzeichnet, daß diese enzymatische Aktivität gemessen wird durch Färbungsmittels eines kolorimetrischen Substrats für diese Säurephosphatase.

10. Verfahren nach Anspruch 9, dadurch gekennzeichnet, daß dieses Substrat alpha-Naphthylphosphat enthält.

11. Verfahren nach Anspruch 6, dadurch gekennzeichnet, daß der Komplex dadurch gebildet wird, daß man diese Säurephosphatase mit Anti-Prostatasäurephosphatase-Antikörpern reagieren läßt, die an einen wasserunlöslichen Träger kovalent gebunden sind.

12. Verfahren nach Anspruch 11, dadurch gekennzeichnet, daß das Vorhandensein dieses Komplexes festgestellt wird durch Messung seiner Säurephosphataseenzymaktivität.

13. Verfahren nach Anspruch 12, dadurch gekennzeichnet, daß diese enzymatische Aktivität gemessen wird durch Färbung mit einem kolorimetrischen Substrat für diese Säurephosphatase.

14. Verfahren nach Anspruch 13, dadurch gekennzeichnet, daß dieses Substrat bei der Reaktion mit diesem Enzym ein fluoreszenzerzeugendes Hydrolyseprodukt bildet, und daß das Vorhandensein dieses Hydrolyseprodukts spektrophotometrisch festgestellt wird.

15. Verfahren nach Anspruch 14, dadurch gekennzeichnet, daß dieses Substrat alpha-Naphthylphosphat enthält.

14